(19) Europäisches Patentamt
European Patent Office
Office européen des brevets

(11) Veröffentlichungsnummer: **0 507 172 A1**

(12) # EUROPÄISCHE PATENTANMELDUNG

(21) Anmeldenummer: **92104972.2**

(22) Anmeldetag: **23.03.92**

(51) Int. Cl.5: **C07D 239/52**, C07D 239/47, C07D 239/42, C07D 251/46, C07D 251/42, A01N 47/44

(30) Priorität: **04.04.91 DE 4110882**

(43) Veröffentlichungstag der Anmeldung: **07.10.92 Patentblatt 92/41**

(84) Benannte Vertragsstaaten: **BE CH DE FR GB IT LI NL**

(71) Anmelder: **BAYER AG**

**W-5090 Leverkusen 1 Bayerwerk(DE)**

(72) Erfinder: **Riebel, Hans-Jochem, Dr.**
**In der Beek 92**
**W-5600 Wuppertal 1(DE)**
Erfinder: **Fest, Christa, Dr.**
**Im Johannistal 20**
**W-5600 Wuppertal 1(DE)**
Erfinder: **Gesing, Ernst R. F., Dr.**
**Trillser Graben 4**
**W-4006 Erkrath-Hochdal(DE)**
Erfinder: **Kirsten, Rolf, Dr.**
**Carl-Langhans-Strasse 27**
**W-4019 Monheim(DE)**
Erfinder: **Kluth, Joachim, Dr.**
**Tannenweg 9**
**W-4018 Langenfeld(DE)**
Erfinder: **Müller, Klaus-Helmut, Dr.**
**Bockhackstrasse 55**
**W-4000 Düsseldorf 13(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**

(54) **Sulfonylguanidinoazine.**

(57) Die Erfindung betrifft neue Sulfonylguanidinoazine der allgemeinen Formel (I)

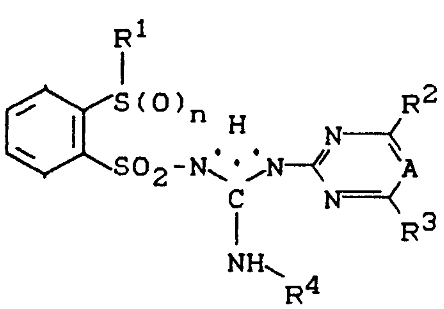

(I)

in welcher

| | |
|---|---|
| n | für die Zahlen 1 oder 2 steht, |
| A | für Stickstoff oder eine C-X-Gruppierung steht, wobei X für Wasserstoff oder Halogen steht, |
| $R^1$ | für Alkyl mit mindestens zwei Kohlenstoffatomen steht, |
| $R^2$ und $R^3$ | gleich oder verschieden sind und für Wasserstoff, Halogen, Alkyl, Halogenalkyl, Alkoxyalkyl, Alkoxy, Halogenalkoxy, Alkylthio, Alkylamino oder Dialkylamino stehen, und |

EP 0 507 172 A1

$R^4$ für Amino oder die Gruppierung

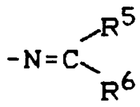

steht, worin

$R^5$ für Wasserstoff oder für jeweils gegebenenfalls substituiertes Alkyl, Aryl oder Aralkyl steht,

$R^6$ für Wasserstoff oder für jeweils gegebenenfalls substituiertes Alkyl, Alkenyl, Alkadienyl, Alkinyl, (Hetero)Aryl, Aralkyl, Aralkenyl, Alkoxy, Alkoxycarbonyl oder Dialkylamino steht, oder zusammen mit $R^5$ für gegebenenfalls substituiertes Alkandiyl steht,

Verfahren zu ihrer Herstellung und ihre Verwendung als Herbizide.

Die Erfindung betrifft neue Sulfonylguanidinoazine, Verfahren zu ihrer Herstellung und ihre Verwendung als Herbizide.

Es ist bekannt, daß bestimmte Sulfonylguanidinoazine, wie z.B. N'-(4,6-Dimethyl-pyrimidin-2-yl)-N''-amino-N'''-(2-diethylaminosulfonyl-phenylsulfonyl)-guanidin, herbizide Eigenschaften aufweisen (vgl. EP-A 173319; vgl. auch EP-A 121082, EP-A 343462 und US-P 4725303). Die herbizide Wirkung der bisher bekannten Sulfonylguanidinoazine ist jedoch nicht immer ganz zufriedenstellend.

Substituierte Sulfonylamidinohydrazone sind Gegenstand vorgängiger, jedoch nicht vorveröffentlichter Patentanmeldungen (vgl. DE-P 3933792/LeA 27241 vom 10.10.1989 und DE-P 4017460/LeA 27629 vom 31.05.1990).

Es wurden nun neue Sulfonylguanidinoazine der allgemeinen Formel (I)

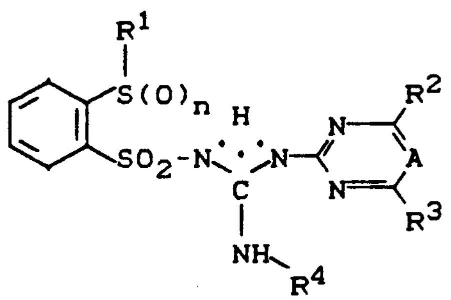

(I)

in welcher

n für die Zahlen 1 oder 2 steht,

A für Stickstoff oder eine C-X-Gruppierung steht, wobei X für Wasserstoff oder Halogen steht,

$R^1$ für Alkyl mit mindestens zwei Kohlenstoffatomen steht,

$R^2$ und $R^3$ gleich oder verschieden sind und für Wasserstoff, Halogen, Alkyl, Halogenalkyl, Alkoxyalkyl, Alkoxy, Halogenalkoxy, Alkylthio, Alkylamino oder Dialkylamino stehen,

und

$R^4$ für Amino oder die Gruppierung

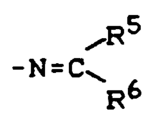

steht,

worin

$R^5$ für Wasserstoff oder für jeweils gegebenenfalls substituiertes Alkyl, Aryl oder Aralkyl steht,

$R^6$ für Wasserstoff oder für jeweils gegebenenfalls substituiertes Alkyl, Alkenyl, Alkadienyl, Alkinyl, (Hetero)Aryl, Aralkyl, Aralkenyl, Alkoxy, Alkoxycarbonyl oder Dialkylamino steht, oder zusammen mit $R^5$ für gegebenenfalls substituiertes Alkandiyl steht,

gefunden.

Die allgemeine Formel (I) steht für die einzelnen möglichen Tautomeren der Formeln (IA), (IB) und (IC)

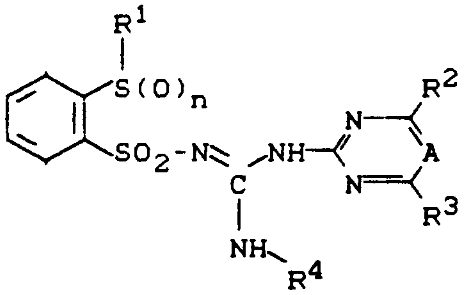

(IA)

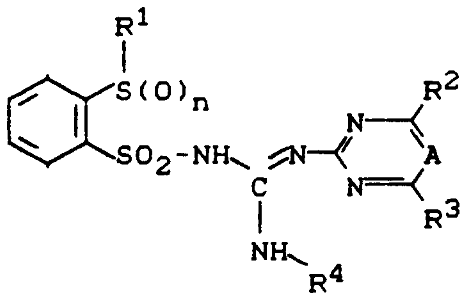

(IB)

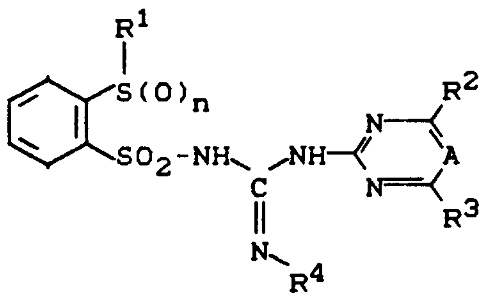

(IC)

und für die Gemische dieser Tautomeren sowie für die E- und Z-Isomeren, welche durch die jeweils vorhandenen C-N-Doppelbindungen bedingt sind, und deren Gemische.

Man erhält die neuen Sulfonylguanidinoazine der allgemeinen Formel (I), wenn man

(a) für den Fall, daß $R^4$ für Amino steht und n, A, $R^1$, $R^2$ und $R^3$ die oben angegebenen Bedeutungen haben,

Sulfonyliso(thio)harnstoffe der allgemeinen Formel (II)

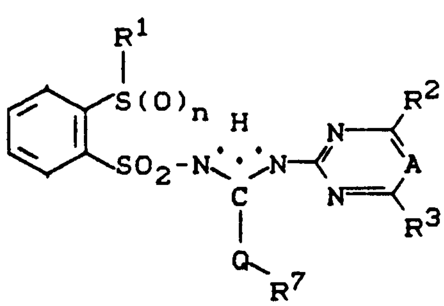

(II)

in welcher

n, A, $R^1$, $R^2$ und $R^3$ die oben angegebenen Bedeutungen haben,

Q für Sauerstoff oder Schwefel steht und

$R^7$ für jeweils gegebenenfalls substituiertes Alkyl, Aralkyl oder Aryl steht,

mit Hydrazin oder einem Hydrazin-Wasser- oder Hydrazin-Säure-Addukt, gegebenenfalls in Gegenwart eines Säureakzeptors und gegebenenfalls in Gegenwart eines Verdünnungsmittels umsetzt, oder wenn

man
(b) für den Fall, daß $R^4$ für die Gruppierung

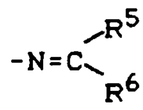

steht und n, A, $R^1$, $R^2$, $R^3$, $R^5$ und $R^6$ die oben angegebenen Bedeutungen haben,
Sulfonylguanidinoazine der allgemeinen Formel (I), in welcher $R^4$ für Amino steht und n, A, $R^1$, $R^2$ und $R^3$ die oben angegebenen Bedeutungen haben,
mit Carbonylverbindungen der allgemeinen Formel (III)

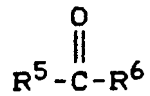 (III)

in welcher
$R^5$ und $R^6$ die oben angegebenen Bedeutungen haben,
oder mit N,N-Dialkyl-carbonsäureamid-acetalen bzw. -ketalen
gegebenenfalls in Gegenwart eines Kondensationshilfsmittels und gegebenenfalls in Gegenwart eines Verdünnungsmittels umsetzt.

Die neuen Sulfonylguanidinoazine der Formel (I) zeichnen sich durch starke herbizide Wirksamkeit aus.

Überraschenderweise zeigen die neuen Sulfonylguanidinoazine der allgemeinen Formel (I) erheblich stärkere herbizide Wirkung als vorbekannte Verbindungen ähnlicher Struktur und gleichartiger Wirkungsrichtung, wie z.B. N'-(4,6-Dimethyl-pyrimidin-2-yl)-N''-amino-N'''-(2-diethylaminosulfonyl-phenylsulfonyl)-guanidin (vgl. EP-A 173319).

Die Erfindung betrifft vorzugsweise neue Sulfonylguanidinoazine der Formel (I), in welcher

n für die Zahlen 1 oder 2 steht,

A für Stickstoff oder eine C-X-Gruppierung steht, wobei X für Wasserstoff, Fluor oder Chlor steht,

$R^1$ für $C_2$-$C_6$-Alkyl steht,

$R^2$ und $R^3$ gleich oder verschieden sind und für Wasserstoff, Fluor, Chlor, Brom, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_4$-Alkylthio, $C_1$-$C_4$-Alkylamino oder Di-($C_1$-$C_2$-alkyl)-amino stehen, und

$R^4$ für Amino oder die Gruppierung

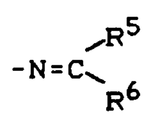

steht, worin

$R^5$ für Wasserstoff, für gegebenenfalls durch Fluor, Chlor und/oder Brom substituiertes $C_1$-$C_6$-Alkyl oder für jeweils gegebenenfalls durch Fluor, Chlor, Brom, $C_1$-$C_4$-Alkyl und/oder $C_1$-$C_4$-Alkoxy substituiertes Phenyl oder Benzyl steht,

$R^6$ für Wasserstoff, für jeweils gegebenenfalls durch Fluor, Chlor und/oder Brom substituiertes $C_1$-$C_{20}$-Alkyl, $C_2$-$C_{10}$-Alkenyl, $C_2$-$C_{10}$-Alkinyl oder $C_4$-$C_{10}$-Alkadienyl, für $C_1$-$C_4$-Alkoxy-$C_1$-$C_4$-alkyl, für $C_1$-$C_4$-Alkylthio-$C_1$-$C_4$-alkyl, für gegebenenfalls durch Fluor, Chlor, Brom, Carboxy, Cyano, Nitro, Amino, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), $C_1$-$C_4$-Alkylthio (welches gegebenenfalls durch Fluor und/oder Chlor substituiert ist), Di-($C_1$-$C_4$-alkyl)-amino, $C_1$-$C_4$-Alkoxy-carbonyl und/oder Phenoxy (welches gegebenenfalls durch Fluor, Chlor und/oder Trifluormethyl substituiert ist) substituiertes Phenyl, für Naphthyl, für jeweils gegebenenfalls durch Cyano, Nitro, Fluor, Chlor, Brom, $C_1$-$C_4$-Alkyl und/oder $C_1$-$C_4$-Alkoxy substituiertes Pyridyl, Pyrrolyl, Furyl, Thiazolyl

oder Thienyl, für Dithienyl, für jeweils gegebenenfalls durch Fluor, Chlor, Brom, $C_1$-$C_4$-Alkyl und/oder $C_1$-$C_4$-Alkoxy substituiertes Phenyl-$C_1$-$C_2$-alkyl oder Phenylethenyl, für $C_1$-$C_6$-Alkoxy, $C_1$-$C_6$-Alkoxy-carbonyl oder Di-($C_1$-$C_4$-alkyl)-amino steht, oder zusammen mit $R^5$ für gegebenenfalls durch Fluor, Chlor, Brom, $C_1$-$C_4$-Alkyl und/oder $C_1$-$C_4$-Alkoxycarbonyl substituiertes $C_2$-$C_6$-Alkandiyl steht.

Die Erfindung betrifft insbesondere Verbindungen der Formel (I), in welcher

n für die Zahlen 1 oder 2 steht,

A für Stickstoff oder eine CH-Gruppierung steht,

$R^1$ für Ethyl, Propyl, Isopropyl, Butyl, Isobutyl oder sec-Butyl steht,

$R^2$ für Wasserstoff, Chlor, Methyl, Ethyl, Trifluormethyl, Methoxymethyl, Methoxy, Ethoxy, Difluormethoxy, Methylthio, Methylamino, Ethylamino oder Dimethylamino steht,

$R^3$ für Wasserstoff, Chlor, Methyl, Ethyl, Trifluormethyl, Methoxy, Ethoxy, Difluormethoxy, Methylthio, Methylamino, Ethylamino oder Dimethylamino steht, und

$R^4$ für Amino oder die Gruppierung

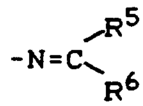

steht, worin

$R^5$ für Wasserstoff, $C_1$-$C_4$-Alkyl oder Phenyl steht,

$R^6$ für $C_1$-$C_{10}$-Alkyl, $C_2$-$C_{10}$-Alkenyl oder $C_4$-$C_{10}$-Alkadienyl, für gegebenenfalls durch Fluor, Chlor, Brom, Carboxy, Cyano, Nitro, Methyl, Ethyl, Trifluormethyl, Methoxy, Ethoxy, Difluormethoxy, Trifluormethoxy, Methylthio, Ethylthio, Difluormethylthio, Trifluormethylthio, Dimethylamino und/oder Phenoxy (welches gegebenenfalls durch Fluor, Chlor und/oder Trifluormethyl substituiert ist) substituiertes Phenyl, für jeweils gegebenenfalls durch Cyano, Nitro, Fluor, Chlor, Brom, Methyl, Ethyl, Methoxy oder Ethoxy substituiertes Pyridyl, Furyl, Thiazolyl oder Thienyl, für Dithienyl, für Benzyl oder Phenylethenyl, für $C_1$-$C_4$-Alkoxy, $C_1$-$C_4$-Alkoxycarbonyl oder Dimethylamino, oder zusammen mit $R^5$ für Butan-1,4-diyl (Tetramethylen) oder Pentan-1,5-diyl (Pentamethylen) steht.

Verwendet man beispielsweise N'-(4,6-Dimethoxy-s-triazin-2-yl)-N''-(2-propylsulfonyl-phenylsulfonyl)-S-methyl-isothioharnstoff und Hydrazin als Ausgangsstoffe, so kann der Reaktionsablauf beim erfindungsgemäßen Verfahren (a) durch das folgende Formelschema skizziert werden:

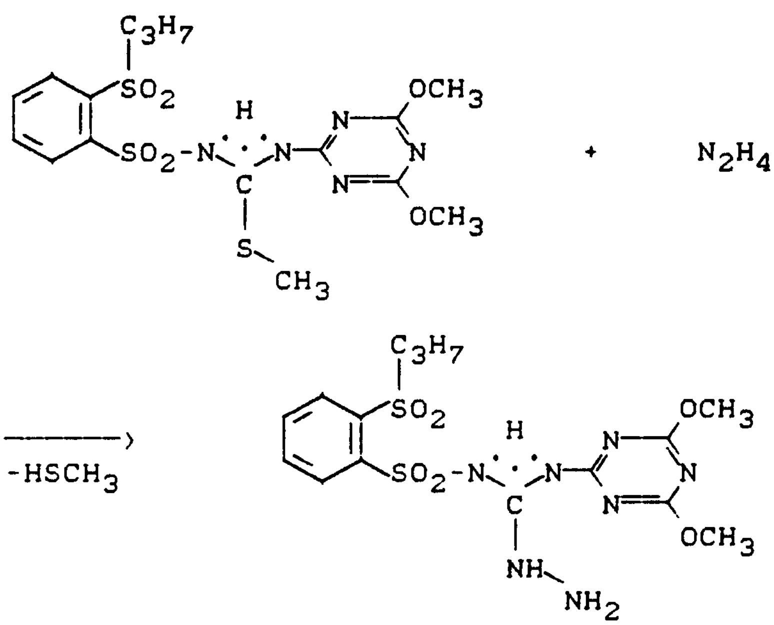

Verwendet man beispielsweise N'-(4,6-Dimethoxy-pyrimidin-2-yl)-N''-amino-N'''-(2-ethylsulfinyl-phenylsulfonyl)-guanidin und Aceton als Ausgangsstoffe, so kann der Reaktionsverlauf beim erfindungsgemäßen Verfahren (b) durch das folgende Formelschema skizziert werden:

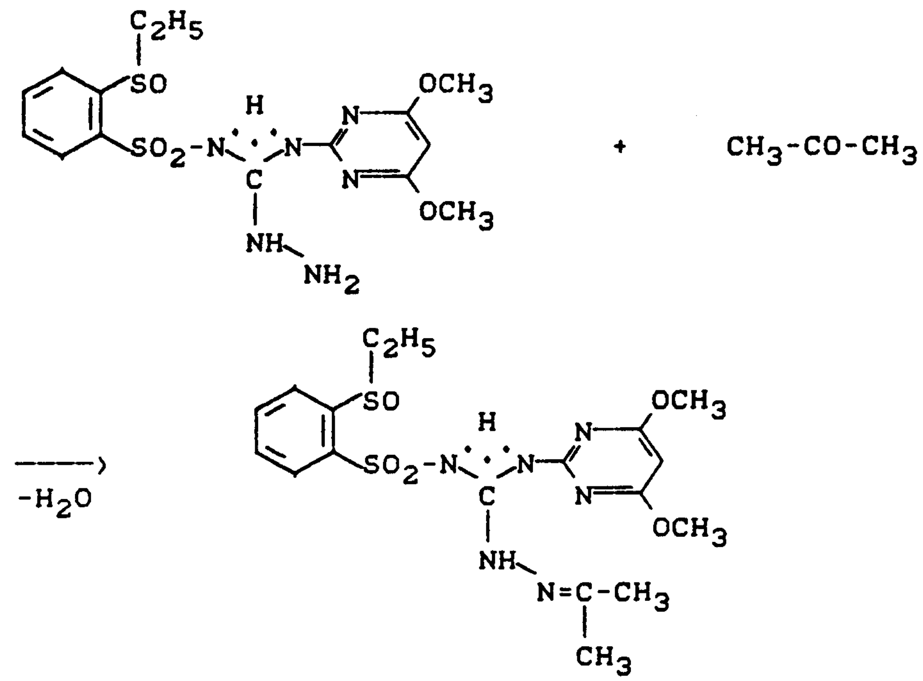


Die beim erfindungsgemäßen Verfahren (a) zur Herstellung von Verbindungen der Formel (I) als Ausgangsstoffe zu verwendenden Sulfonyliso(thio)harnstoffe sind durch die Formel (II) allgemein definiert.

In Formel (II) haben n, A, $R^1$, $R^2$ und $R^3$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, A, $R^1$, $R^2$ und $R^3$ angegeben wurden;

Q steht vorzugsweise für Sauerstoff oder Schwefel und

$R^7$ steht vorzugsweise für gegebenenfalls durch Carboxy, $C_1$-$C_4$-Alkoxycarbonyl oder $C_1$-$C_4$-Alkoxy substituiertes $C_1$-$C_4$-Alkyl, insbesondere für Methyl, oder für jeweils gegebenenfalls durch Fluor, Chlor, Brom, $C_1$-$C_4$-Alkyl oder $C_1$-$C_4$-Alkoxy substituiertes Benzyl oder Phenyl.

Die Ausgangsstoffe der Formel (II) sind noch nicht aus der Literatur bekannt und sind Gegenstand einer parallel eingereichten Patentanmeldung.

Man erhält die Sulfonyliso(thio)harnstoffe der Formel (II), wenn man entsprechende Verbindungen der allgemeinen Formel (IV)

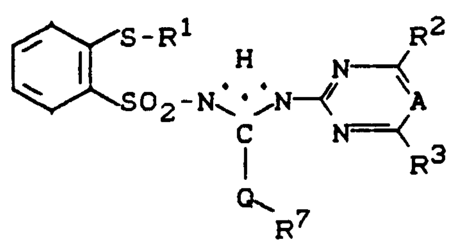

(IV)

in welcher

n, A, $R^1$, $R^2$, $R^3$, Q und $R^7$ die oben angegebenen Bedeutungen haben,

mit einem Oxidationsmittel, wie z.B. Hydrogenperoxid, gegebenenfalls in Gegenwart eines Verdünnungsmittels, wie z.B. Essigsäure, bei Temperaturen zwischen 0 °C und 100 °C umsetzt.

Die Verbindungen der Formel (IV) sind bekannt und/oder können nach an sich bekannten Verfahren hergestellt werden (vgl. EP-A 413221).

Das erfindungsgemäße Verfahren (a) wird unter Einsatz von Hydrazin oder einem Hydrazin-Wasser-

oder Hydrazin-Säure-Addukt durchgeführt.
Vorzugsweise kommen praktisch wasserfreies Hydrazin oder Hydrazin-Hydrat sowie Hydrazin-Addukte mit Mineralsäuren, wie z. B. Hydrazin-mono- oder -di-hydrochlorid und Hydrazinsulfat in Betracht.

Diese Verbindungen sind bekannte Synthesechemikalien.

Das erfindungsgemäße Verfahren (a) zur Herstellung der neuen Sulfonylguanidinoazine der Formel (I) wird vorzugsweise unter Verwendung von Verdünnungsmitteln durchgeführt. Als Verdünnungsmittel kommen dabei vorzugsweise Wasser und/oder polare organische Lösungsmittel, wie Methanol, Ethanol, Isopropanol, Butanol, Isobutanol, sec-Butanol, tert-Butanol, Glycoldimethylether, Diglycoldimethylether, Tetrahydrofuran, Dioxan, Essigsäuremethylester, Essigsäureethylester, Acetonitril, Propionitril, Dimethylformamid, Dimethylacetamid, N-Methylpyrrolidon, Methylenchlorid, Chloroform, Dimethylsulfoxid und Tetramethylensulfon, in Betracht.

Als Säureakzeptoren können bei dem erfindungsgemäßen Verfahren alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen -20 °C und +80 °C, vorzugsweise bei Temperaturen zwischen 0 °C und 50 °C.

Das erfindungsgemäße Verfahren (a) wird im allgemeinen bei Normaldruck durchgeführt.

Zur Durchführung des erfindungsgemäßen Verfahrens (a) setzt man je Mol Sulfonyliso(thio)harnstoff der Formel (II) im allgemeinen zwischen 1 und 3 Mol, vorzugsweise zwischen 1,0 und 1,5 Mol, Hydrazin bzw. Hydrazin-Wasser-Addukt bzw. Hydrazin-Säure-Addukt ein.

Im allgemeinen werden die Reaktionskomponenten bei Raumtemperatur oder unter Eiskühlung zusammen gegeben und das Reaktionsgemisch wird, gegebenenfalls bei erhöhter Temperatur, bis zum Ende der Umsetzung gerührt. Die Produkte fallen im allgemeinen kristallin an; andernfalls können sie nach Einengen und Verreiben mit einem geeigneten Verdünnungsmittel, wie z.B. Ethanol, kristallin erhalten und durch Absaugen isoliert werden.

Die beim erfindungsgemäßen Verfahren (b) als Ausgangsstoffe zu verwendenden Verbindungen sind durch die Formel (I) mit der Maßgabe, daß $R^4$ für Amino steht, allgemein definiert.
In diesem Fall haben n, A, $R^1$, $R^2$ und $R^3$ vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden.

Die oben beschriebenen Ausgangsstoffe der Formel (I) für Verfahren (b) sind erfindungsgemäße, neue Verbindungen; sie können nach dem erfindungsgemäßen Verfahren (a) hergestellt werden.

Die beim erfindungsgemäßen Verfahren (b) zur Herstellung von Verbindungen der Formel (I) weiter als Ausgangsstoffe zu verwendenden Carbonylverbindungen sind durch die Formel (III) allgemein definiert.

In Formel (III) haben $R^5$ und $R^6$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^5$ und $R^6$ angegeben wurden.

Als Beispiele für die Ausgangsstoffe der Formel (III) seien genannt:
Formaldehyd, Acetaldehyd, Propionaldehyd, Butyraldehyd, Isobutyraldehyd, Valeraldehyd, Isovaleraldehyd, Benzaldehyd, Pyridin-4-, -2- und -3-carbaldehyd, Furan-2- und -3-carbaldehyd sowie Thiophen-2- und -3-carbaldehyd, ferner auch Aceton, Methylethylketon, Methylpropylketon, Methylisopropylketon, Methylbutylketon, Methylisobutylketon, Diethylketon, Acetophenon, Benzophenon, Cyclopentanon, Cyclohexanon, Phenylaceton, Chloraceton, Chloral, Glyoxylsäuremethylester und -ethylester, Brenztraubensäuremethylester und -ethylester, Phthalaldehydsäure und 6-Chlor-pyridin-3-aldehyd.

N,N-Dialkyl-carbonsäureamid-acetale bzw. -ketale, welche weiter als Ausgangsstoffe beim erfindungsgemäßen Verfahren (b) eingesetzt werden können, sind vorzugsweise N,N-Dialkyl-formamid- oder - acetamid-acetale bzw. -ketale mit an N- bzw. O gebundenen geradkettigen oder verzweigten Alkylresten mit 1 bis 4 Kohlenstoffatomen, wie z.B. N,N-Dimethyl- und N,N-Diethyl-formamid- und -acetamid-dimethylacetal, -diethylacetal, -dipropylacetal, -diisopropylacetal, -dibutylacetal, -diisobutylacetal, -di-sec-butyl-acetal und -di-tert-butyl-acetal.

Die Ausgangsstoffe der Formel (III) sind bekannte organische Synthesechemikalien.

Das erfindungsgemäße Verfahren (b) zur Herstellung der neuen Verbindungen der Formel (I) wird gegebenenfalls unter Verwendung von Verdünnungsmitteln durchgeführt. Als Verdünnungsmittel kommen

dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Alkohole wie Methanol, Ethanol, Propanol, Isopropanol, Butanol und Isobutanol, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Das erfindungsgemäße Verfahren (b) wird gegebenenfalls in Gegenwart eines Kondensationshilfsmittels durchgeführt. Als solche kommen vorzugsweise die in der organischen Chemie üblichen Trockenmittel in Betracht. Hierzu gehören vorzugsweise wasserfreie Salze, wie z.B. Natriumsulfat, Magnesiumsulfat und Kaliumcarbonat.

Die Reaktionstemperaturen können bei dem erfindungsgemäßen Verfahren (b) in einem größeren Bereich variiert werden. Im allgemeinen arbeitet man bei Temperaturen zwischen 0 °C und 100 °C, vorzugsweise bei Temperaturen zwischen 10 °C und 80 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) setzt man auf 1 Mol Ausgangsverbindung der Formel (I) im allgemeinen zwischen 1 und 1000 Mol, vorzugsweise zwischen 1 und 500 Mol Carbonylverbindung der Formel (III) ein.

Die Reaktionskomponenten werden im allgemeinen bei Raumtemperatur oder unter leichtem Kühlen vermischt und gegebenenfalls bei erhöhter Temperatur bis zum Ende der Umsetzung gerührt. Die Aufarbeitung erfolgt nach üblichen Methoden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); Chloressigsäure-N-(methoxymethyl)-2,6-diethylanilid (ALACHLOR); Methyl-6,6-dimethyl-2,4-dioxo-3-[1-(2-propenyloxyamino)-butyliden]-cyclohexancarbonsäure (ALLOXYDIM); 4-Amino-benzolsulfonyl-methylcarbamat (ASULAM); 2-Chlor-4-ethylamino-6-isopropylamino-1,3,5-triazin (ATRAZIN); 2-[[[[(4,6-Dimethoxypyrimidin-2-yl)-amino]-carbonyl]-amino]-sulfonyl]-methyl]-benzoesäuremethylester (BENSULFURON); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxybenzonitril (BROMOXYNIL); N-(Butoxymethyl)-2-chlor-N-(2,6-diethylphenyl)-acetamid(BUTACHLOR); Ethyl-2-{[(4-chlor-6-methoxy-2-pyrimidinyl)-aminocarbonyl]-aminosulfonyl}-benzoat (CHLORIMURON); N-(3-Chlorphenyl)-isopropylcarbamat (CHLORPROPHAM); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); exo-1-Methyl-4-(1-methylethyl)-2-(2-methylphenyl-methoxy)-7-oxabicyclo-(2,2,1)-heptan (CINMETHYLIN); 3,6-Dichlor-2-pyridincarbonsäure (CLOPYRALID); 2-Chlor-4-ethylamino-6-(3-cyanopropylamino)-1,3,5-triazin (CYANAZIN); N,S-Diethyl-N-cyclohexyl-thiolcarbamat (CYCLOATE); 2-[1-(Ethoximino)-butyl]-3-hydroxy-5-[tetrahydro-(2H)-thiopyran-3-yl]-2-cyclohexen-1-on (CYCLOXYDIM); 2-[4-(2,4-Dichlorphenoxy)-phenoxy]-propionsäure, deren Methyl- oder deren Ethylester (DICLOFOP); 2-[(2-Chlorphenyl)-methyl]-4,4-dimethylisoxazolidin-3-on (DIMETHAZONE); N,N-Di-n-propyl-thiocarbamidsäure-S-ethylester (EPTAME); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); 2-[4-(5-Trifluormethyl-2-pyridyloxy)-phenoxyl-propansäure oder deren Butylester (FLUAZIFOP); N,N-Dimethyl-N'-(3-trifluormethylphenyl)-harnstoff (FLUOMETURON); 1-Methyl-3-phenyl-5-(3-trifluormethylphenyl)-4-pyridon (FLURIDONE); [(4-Amino-3,5-dichlor-6-fluor-2-pyridinyl)-oxy]-essigsäure bzw. deren 1-Methylheptylester (FLUROXYPYR); 2-{4-[(3-Chlor-5-(trifluormethyl)-2-pyridinyl)-oxy]-phenoxy}-propansäure bzw. deren Ethylester (HALOXYFOP); 3-Cyclohexyl-6-dimethylamino-1-methyl-1,3,5-triazin-2,4-dion (HEXAZINONE); Methyl-2-[4,5-dihydro-4-methyl-4-(1-methylethyl)-5-oxo-1H-imidazol-2-yl]-4(5)-methylbenzoat (IMAZAMETHABENZ); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); 3,5-Diiod-4-hydroxybenzonitril (IOXYNIL); N, N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); S-Ethyl-N,N-hexamethylen-thiolcarbamat (MOLINATE); 1-(3-Trifluormethyl-phenyl)-4-methylamino-5-chlor-6-pyridazon (NORFLURAZON); 4-(Di-n-propylamino)-3,5-dinitrobenzolsulfonamid (ORYZALIN); (2-Chlor-4-trifluormethylphenyl)-(3-ethoxy-4-nitro-phenyl)-ether (OXYFLUORFEN); N-(1-Ethylpropyl)-3,4-dimethyl-2,6-dinitroanilin (PENDIMETHALIN); α-Chlor-2',6'-diethyl-N-(2-propoxyethyl)-acetanilid (PRETILACHLOR); 2-Chlor-N-isopropylacetanilid (PROPACHLOR); Isopropyl-N-phenylcarbamat (PROPHAM); 0-(6-Chlor-3-phenyl-pyridazin-4-yl)-S-octyl-thiocarbonat (PYRIDATE); 2-[4-(6-Chlor-chinoxalin-2-yl-oxy)-phenoxy]-propionsäure-ethylester (QUIZALOFOPETHYL); 2-[1-(Ethoxamino)-butyliden]-5-(2-ethylthiopropyl)-1,3-cyclohexadion (SETHOXYDIM); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); S-[(4-Chlorphenyl)-methyl]-N,N-diethyl-thiocarbamat (THIOBENCARB); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,5 g und 1 kg Wirkstoff pro Hektar Bodenfläche, vorzugsweise zwischen 1 g und 500 g pro ha.

Die Herstellung und die Verwendung der erfindungsgemäßen Wirkstoffe geht aus den nachfolgenden Beispielen hervor.

Herstellungsbeispiele:

Beispiel 1

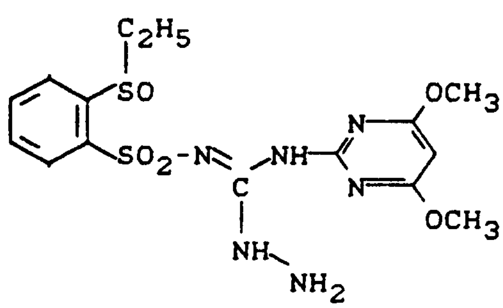

(Verfahren (a))

Eine Mischung aus 13,3 g (0,03 Mol) N'-(4,6-Dimethoxy-pyrimidin-2-yl)-N''-(2-ethylsulfinyl-phenylsulfonyl)-S-methyl-isothioharnstoff, 2 ml (0,04 Mol $N_2H_4$) Hydrazinhydrat und 70 ml Chloroform wird 60 Minuten bei 20 °C gerührt, dann mit Natriumsulfat getrocknet und filtriert. Das Filtrat wird eingeengt, der Rückstand mit Ethanol verrieben und das kristallin angefallene Produkt durch Absaugen isoliert.

Man erhält 9,5 g (74% der Theorie) N'-(4,6-Dimethoxy-pyrimidin-2-yl)-N''-amino-N'''-(2-ethylsulfinyl-phenylsulfonyl)-guanidin vom Schmelzpunkt 134 °C.

Beispiel 2

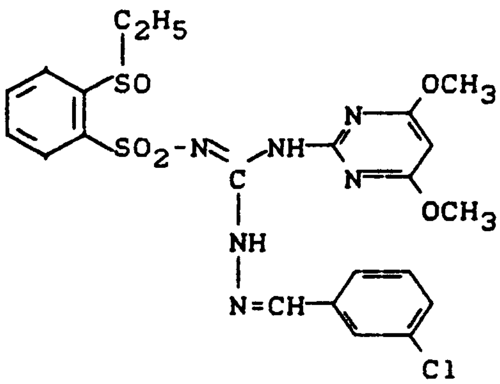

(Verfahren (b))

Eine Mischung aus 3,4 g (8 mMol) N'-(4,6-Dimethoxy-pyrimidin-2-yl)-N''-amino-N'''-(2-ethylsulfinyl-phenylsulfonyl)-guanidin, 1,3 g (9 mMol) 3-Chlor-benzaldehyd und 40 ml Ethanol wird 2 Stunden bei 70 °C gerührt. Nach dem Erkalten wird das kristallin angefallene Produkt durch Absaugen isoliert.

Man erhält 4,0 g (91% der Theorie) N'-(4,6-Dimethoxy-pyrimidin-2-yl)-N''-(3-chlor-benzylidenamino)-N'''-(2-ethylsulfinyl-phenylsulfonyl)-guanidin vom Schmelzpunkt 184 °C.

Analog zu den Beispielen 1 und 2 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

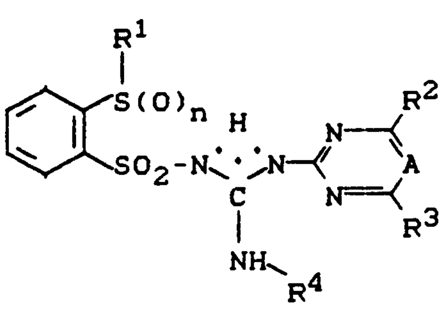

(I)

**Tabelle 1:** Beispiele für die Verbindungen der Formel (I)

| Bsp.-Nr. | n | A | $R^1$ | $R^2$ | $R^3$ | $R^4$ | Schmelzpunkt (°C) |
|---|---|---|---|---|---|---|---|
| 3 | 1 | CH | $C_2H_5$ | $OCH_3$ | $OCH_3$ | 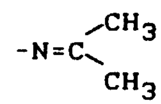 | 184 |
| 4 | 1 | CH | $C_2H_5$ | $OCH_3$ | $OCH_3$ | 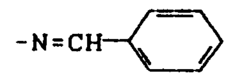 | 187 |
| 5 | 1 | CH | $C_2H_5$ | $OCH_3$ | $OCH_3$ | 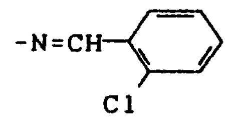 | 193 |
| 6 | 1 | CH | $C_2H_5$ | $OCH_3$ | $OCH_3$ | 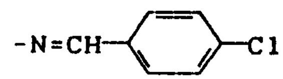 | 208 |
| 7 | 1 | CH | $C_2H_5$ | $OCH_3$ | $OCH_3$ | 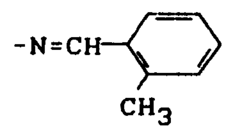 | 191 |
| 8 | 1 | CH | $C_2H_5$ | $OCH_3$ | $OCH_3$ | 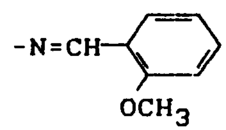 | 200 |
| 9 | 1 | CH | $C_2H_5$ | $OCH_3$ | $OCH_3$ | 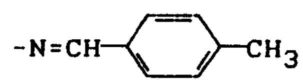 | 205 |

<u>Tabelle 1</u> - Fortsetzung

| Bsp.-Nr. | n | A | $R^1$ | $R^2$ | $R^3$ | $R^4$ | Schmelzpunkt (°C) |
|---|---|---|---|---|---|---|---|
| 10 | 1 | CH | $C_2H_5$ | $OCH_3$ | $OCH_3$ | 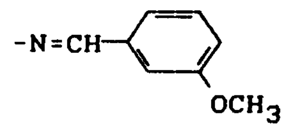 | 155 |
| 11 | 1 | CH | $C_2H_5$ | $OCH_3$ | $OCH_3$ | 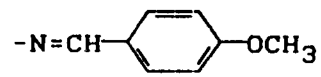 | 215 |
| 12 | 1 | CH | $C_2H_5$ | $OCH_3$ | $OCH_3$ | 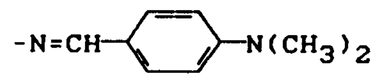 | 209 |
| 13 | 1 | CH | $C_3H_7$ | $OCH_3$ | $OCH_3$ | $NH_2$ | (amorph) |
| 14 | 1 | N | $C_2H_5$ | $OCH_3$ | $OCH_3$ | $NH_2$ | 112 |
| 15 | 1 | N | $C_2H_5$ | $OCH_3$ | $OCH_3$ | 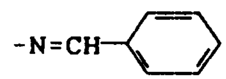 | 166 |
| 16 | 1 | N | $C_2H_5$ | $OCH_3$ | $OCH_3$ | 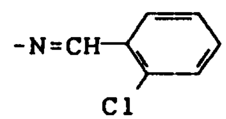 | 175 |
| 17 | 1 | N | $C_2H_5$ | $OCH_3$ | $OCH_3$ | 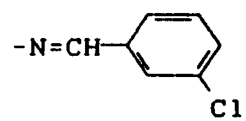 | 204 |

**Tabelle 1** - Fortsetzung

| Bsp.-Nr. | n | A | $R^1$ | $R^2$ | $R^3$ | $R^4$ | Schmelzpunkt (°C) |
|---|---|---|---|---|---|---|---|
| 18 | 1 | N | $C_2H_5$ | $OCH_3$ | $OCH_3$ | -N=CH- 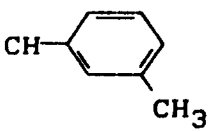 | 128 |
| 19 | 1 | N | $C_2H_5$ | $OCH_3$ | $OCH_3$ | -N=CH- 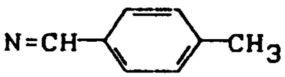 | 197 |
| 20 | 1 | N | $C_2H_5$ | $OCH_3$ | $OCH_3$ | -N=CH- 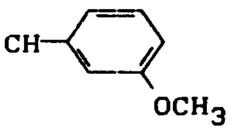 | 126 |
| 21 | 1 | N | $C_2H_5$ | $OCH_3$ | $OCH_3$ | -N=CH- 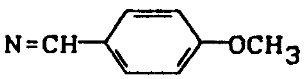 | 189 |
| 22 | 2 | CH | $C_2H_5$ | $OCH_3$ | $OCH_3$ | $NH_2$ | 187 |
| 23 | 2 | CH | $C_2H_5$ | $OCH_3$ | $OCH_3$ | 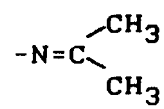 | 209 |
| 24 | 2 | CH | $C_2H_5$ | $OCH_3$ | $OCH_3$ | -N=CH- 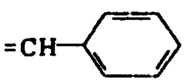 | 221 |

<u>**Tabelle 1**</u> - Fortsetzung

| Bsp.-Nr. | n | A | $R^1$ | $R^2$ | $R^3$ | $R^4$ | Schmelzpunkt (°C) |
|---|---|---|---|---|---|---|---|
| 25 | 2 | CH | $C_2H_5$ | $OCH_3$ | $OCH_3$ | -N=CH- 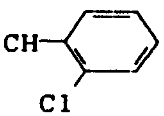 | 209 |
| 26 | 2 | CH | $C_2H_5$ | $OCH_3$ | $OCH_3$ | -N=CH- 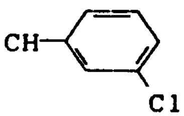 | 221 |
| 27 | 2 | CH | $C_2H_5$ | $OCH_3$ | $OCH_3$ | -N=CH- 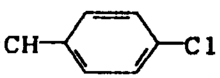 | 229 |
| 28 | 2 | CH | $C_2H_5$ | $OCH_3$ | $OCH_3$ | -N=CH- 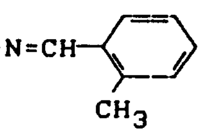 | 199 |
| 29 | 2 | CH | $C_2H_5$ | $OCH_3$ | $OCH_3$ | -N=CH- 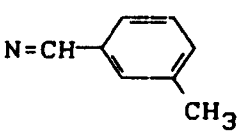 | 224 |
| 30 | 2 | CH | $C_2H_5$ | $OCH_3$ | $OCH_3$ | -N=CH- 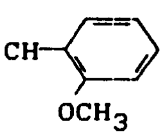 | 215 |

<u>**Tabelle 1**</u> - Fortsetzung

| Bsp.-Nr. | n | A | $R^1$ | $R^2$ | $R^3$ | $R^4$ | Schmelzpunkt (°C) |
|---|---|---|---|---|---|---|---|
| 31 | 2 | CH | $C_2H_5$ | $OCH_3$ | $OCH_3$ | 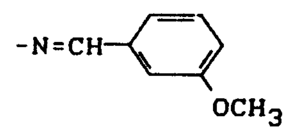 | 215 |
| 32 | 2 | CH | $C_2H_5$ | $OCH_3$ | $OCH_3$ | 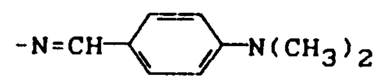 | 256 |
| 33 | 2 | N | $C_2H_5$ | $OCH_3$ | $OCH_3$ | $NH_2$ | 142 |
| 34 | 2 | N | $C_2H_5$ | $OCH_3$ | $OCH_3$ | 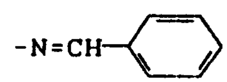 | 199 |
| 35 | 2 | N | $C_2H_5$ | $OCH_3$ | $OCH_3$ | 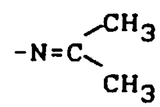 | 195 |
| 36 | 2 | N | $C_2H_5$ | $OCH_3$ | $OCH_3$ | 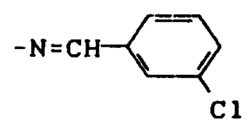 | 201 |
| 37 | 2 | N | $C_2H_5$ | $OCH_3$ | $OCH_3$ | 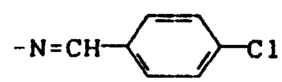 | 212 |

<u>Tabelle 1</u> - Fortsetzung

| Bsp.-Nr. | n | A | $R^1$ | $R^2$ | $R^3$ | $R^4$ | Schmelzpunkt (°C) |
|---|---|---|---|---|---|---|---|
| 38 | 2 | N | $C_2H_5$ | $OCH_3$ | $OCH_3$ | 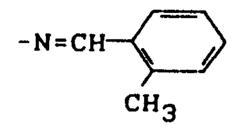 | 190 |
| 39 | 2 | N | $C_2H_5$ | $OCH_3$ | $OCH_3$ | 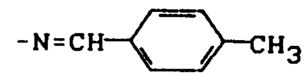 | 214 |
| 40 | 2 | N | $C_2H_5$ | $OCH_3$ | $OCH_3$ | 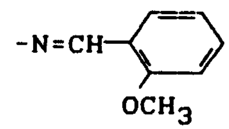 | 209 |
| 41 | 2 | N | $C_2H_5$ | $OCH_3$ | $OCH_3$ | 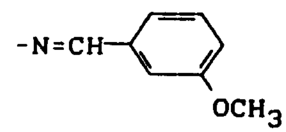 | 201 |
| 42 | 2 | N | $C_2H_5$ | $OCH_3$ | $OCH_3$ | 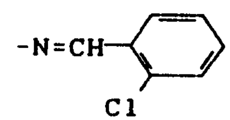 | 209 |
| 43 | 2 | N | $C_2H_5$ | $OCH_3$ | $OCH_3$ | 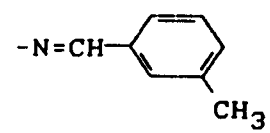 | 187 |

<u>Tabelle 1</u> - Fortsetzung

| Bsp.-Nr. | n | A | $R^1$ | $R^2$ | $R^3$ | $R^4$ | Schmelzpunkt (°C) |
|---|---|---|---|---|---|---|---|
| 44 | 2 | N | $C_2H_5$ | $CH_3$ | $OCH_3$ | $NH_2$ | 149 |
| 45 | 2 | N | $C_2H_5$ | $CH_3$ | $OCH_3$ | 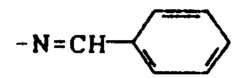 | 192 |
| 46 | 2 | N | $C_2H_5$ | $CH_3$ | $OCH_3$ | 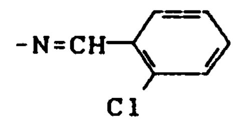 | 207 |
| 47 | 1 | N | $C_2H_5$ | $CH_3$ | $OCH_3$ | $NH_2$ | 87 |
| 48 | 1 | N | $C_2H_5$ | $CH_3$ | $OCH_3$ | 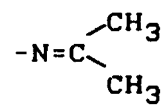 | 201 |
| 49 | 1 | N | $C_2H_5$ | $CH_3$ | $OCH_3$ | 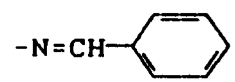 | 153 |
| 50 | 1 | N | $C_2H_5$ | $CH_3$ | $OCH_3$ | 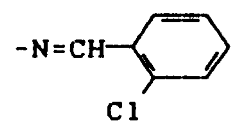 | 152 |

Tabelle 1 - Fortsetzung

| Bsp.-Nr. | n | A | $R^1$ | $R^2$ | $R^3$ | $R^4$ | Schmelzpunkt (°C) |
|---|---|---|---|---|---|---|---|
| 51 | 1 | N | $C_2H_5$ | $CH_3$ | $OCH_3$ | 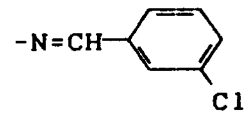 | 87 |
| 52 | 1 | N | $C_2H_5$ | $CH_3$ | $OCH_3$ | 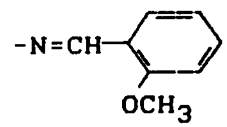 | 183 |
| 53 | 1 | N | $C_2H_5$ | $CH_3$ | $OCH_3$ | 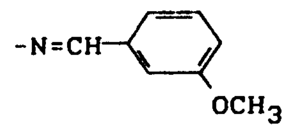 | 168 |
| 54 | 1 | N | $C_2H_5$ | $CH_3$ | $OCH_3$ | 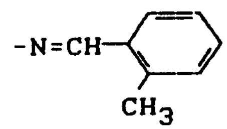 | 173 |
| 55 | 1 | N | $C_2H_5$ | $CH_3$ | $OCH_3$ | 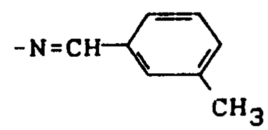 | 157 |
| 56 | 2 | N | $C_2H_5$ | $CH_3$ | $OCH_3$ | 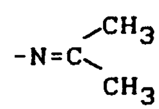 | 215 |

Tabelle 1 - Fortsetzung

| Bsp.-Nr. | n | A | $R^1$ | $R^2$ | $R^3$ | $R^4$ | Schmelzpunkt (°C) |
|---|---|---|---|---|---|---|---|
| 57 | 1 | CH | $C_3H_7$ | $OCH_3$ | $OCH_3$ | 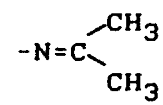 | 169 |
| 58 | 1 | CH | $C_3H_7$ | $OCH_3$ | $OCH_3$ | 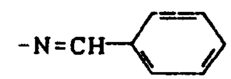 | 155 |
| 59 | 1 | CH | $C_3H_7$ | $OCH_3$ | $OCH_3$ | 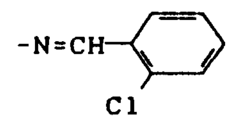 | 182 |
| 60 | 1 | CH | $C_3H_7$ | $OCH_3$ | $OCH_3$ | 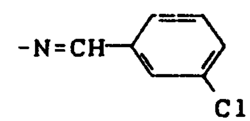 | 185 |
| 61 | 1 | CH | $C_3H_7$ | $OCH_3$ | $OCH_3$ | 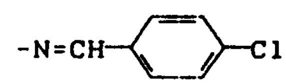 | 185 |
| 62 | 1 | CH | $C_3H_7$ | $OCH_3$ | $OCH_3$ | 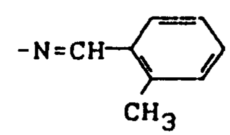 | 172 |

<u>Tabelle 1</u> - Fortsetzung

| Bsp.-Nr. | n | A | $R^1$ | $R^2$ | $R^3$ | $R^4$ | Schmelzpunkt (°C) |
|---|---|---|---|---|---|---|---|
| 63 | 1 | CH | $C_3H_7$ | $OCH_3$ | $OCH_3$ | -N=CH 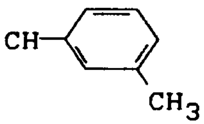 | 186 |
| 64 | 1 | CH | $C_3H_7$ | $OCH_3$ | $OCH_3$ | -N=CH 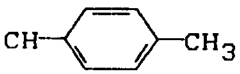 | 178 |
| 65 | 1 | CH | $C_3H_7$ | $OCH_3$ | $OCH_3$ | -N=CH 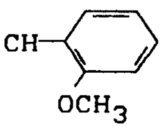 | 188 |
| 66 | 1 | CH | $C_3H_7$ | $OCH_3$ | $OCH_3$ | -N=CH 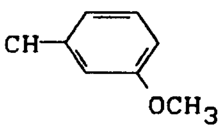 | 172 |
| 67 | 1 | CH | $C_3H_7$ | $OCH_3$ | $OCH_3$ | -N=CH 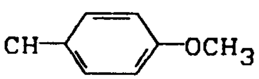 | 193 |
| 68 | 1 | N | $C_3H_7$ | $CH_3$ | $OCH_3$ | $NH_2$ | (amorph) |
| 69 | 1 | N | $C_3H_7$ | $OCH_3$ | $OCH_3$ | $NH_2$ | (amorph) |
| 70 | 2 | CH | $C_3H_7$ | $OCH_3$ | $OCH_3$ | $NH_2$ | |
| 71 | 2 | CH | $C_3H_7$ | $CH_3$ | $OCH_3$ | $NH_2$ | |

Tabelle 1 - Fortsetzung

| Bsp.-Nr. | n | A | $R^1$ | $R^2$ | $R^3$ | $R^4$ | Schmelzpunkt (°C) |
|---|---|---|---|---|---|---|---|
| 72 | 2 | CH | $C_2H_5$ | $CH_3$ | $OCH_3$ | $NH_2$ | |
| 73 | 2 | CH | $C_2H_5$ | H | $CH_3$ | $NH_2$ | |
| 74 | 2 | CH | $C_2H_5$ | $CH_3$ | $CH_3$ | $NH_2$ | |
| 75 | 1 | CH | $C_2H_5$ | $CH_3$ | $OCH_3$ | $NH_2$ | |
| 76 | 1 | CH | $C_2H_5$ | $CH_3$ | $CH_3$ | $NH_2$ | 126 |
| 77 | 1 | CH | $C_2H_5$ | $CH_3$ | H | $NH_2$ | 125 |
| 78 | 2 | N | $C_3H_7$ | $CH_3$ | $OCH_3$ | $NH_2$ | |
| 79 | 2 | CH | $C_3H_7$ | $CH_3$ | $CH_3$ | $NH_2$ | |
| 80 | 2 | CH | $C_3H_7$ | H | $CH_3$ | $NH_2$ | |
| 81 | 1 | CH | $C_3H_7$ | $CH_3$ | $OCH_3$ | $NH_2$ | |
| 82 | 1 | CH | $C_3H_7$ | $CH_3$ | $CH_3$ | $NH_2$ | |
| 83 | 1 | CH | $C_3H_7$ | H | $CH_3$ | $NH_2$ | 86 |
| 84 | 1 | CH | $CH(CH_3)_2$ | $OCH_3$ | $OCH_3$ | $NH_2$ | |
| 85 | 1 | N | $CH(CH_3)_2$ | $OCH_3$ | $OCH_3$ | $NH_2$ | |

Tabelle 1 - Fortsetzung

| Bsp.-Nr. | n | A | $R^1$ | $R^2$ | $R^3$ | $R^4$ | Schmelzpunkt (°C) |
|---|---|---|---|---|---|---|---|
| 86 | 1 | N | $CH(CH_3)_2$ | $CH_3$ | $OCH_3$ | $NH_2$ | |
| 87 | 1 | CH | $CH(CH_3)_2$ | $CH_3$ | $OCH_3$ | $NH_2$ | |
| 88 | 1 | CH | $CH(CH_3)_2$ | $CH_3$ | $CH_3$ | $NH_2$ | |
| 89 | 2 | CH | $CH(CH_3)_2$ | $OCH_3$ | $OCH_3$ | $NH_2$ | |
| 90 | 2 | CH | $CH(CH_3)_2$ | $CH_3$ | $OCH_3$ | $NH_2$ | |
| 91 | 2 | CH | $CH(CH_3)_2$ | $CH_3$ | $CH_3$ | $NH_2$ | |
| 92 | 2 | CH | $CH(CH_3)_2$ | $CH_3$ | H | $NH_2$ | |
| 93 | 2 | N | $CH(CH_3)_2$ | $OCH_3$ | $OCH_3$ | $NH_2$ | |
| 94 | 2 | N | $CH(CH_3)_2$ | $CH_3$ | $OCH_3$ | $NH_2$ | |
| 95 | 2 | N | $CH(CH_3)_2$ | $CH_3$ | $CH_3$ | $NH_2$ | |
| 96 | 1 | N | $C_3H_7$ | $OCH_3$ | $OCH_3$ | 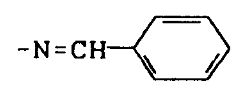 | 118 |
| 97 | 1 | N | $C_3H_7$ | $OCH_3$ | $OCH_3$ | 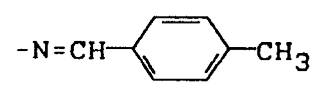 | |

Tabelle 1 - Fortsetzung

| Bsp.-Nr. | n | A | $R^1$ | $R^2$ | $R^3$ | $R^4$ | Schmelzpunkt (°C) |
|---|---|---|---|---|---|---|---|
| 98 | 1 | N | $C_3H_7$ | $OCH_3$ | $OCH_3$ | -N=CH- 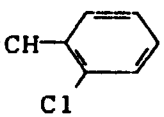 | |
| 99 | 1 | N | $C_3H_7$ | $OCH_3$ | $OCH_3$ | -N=CH- 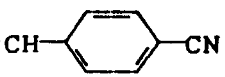 | |
| 100 | 1 | N | $C_3H_7$ | $OCH_3$ | $OCH_3$ | -N=CH- 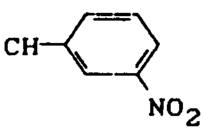 | |
| 101 | 1 | N | $C_3H_7$ | $CH_3$ | $OCH_3$ | -N=CH- 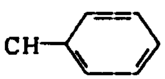 | 169 |
| 102 | 1 | N | $C_3H_7$ | $CH_3$ | $OCH_3$ | -N=CH- 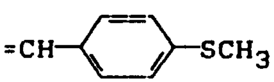 | |
| 103 | 1 | N | $C_3H_7$ | $CH_3$ | $OCH_3$ | -N=CH- 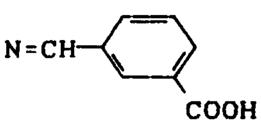 | |
| 104 | 1 | N | $C_3H_7$ | $CH_3$ | $OCH_3$ | -N=CH- 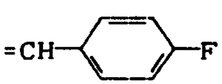 | |

Tabelle 1 - Fortsetzung

| Bsp.-Nr. | n | A | $R^1$ | $R^2$ | $R^3$ | $R^4$ | Schmelzpunkt (°C) |
|---|---|---|---|---|---|---|---|
| 105 | 1 | N | $C_3H_7$ | $CH_3$ | $OCH_3$ | -N=CH- 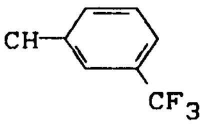 | |
| 106 | 1 | CH | $C_3H_7$ | $CH_3$ | $CH_3$ | -N=CH- 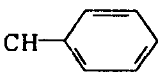 | |
| 107 | 1 | CH | $C_3H_7$ | $CH_3$ | $OCH_3$ | -N=CH- 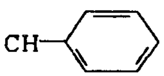 | |
| 108 | 1 | CH | $C_3H_7$ | H | $CH_3$ | -N=CH- 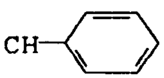 | 135 |
| 109 | 2 | CH | $C_3H_7$ | $OCH_3$ | $OCH_3$ | -N=CH- 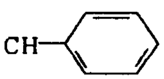 | |
| 110 | 2 | CH | $C_3H_7$ | $OCH_3$ | $OCH_3$ | -N=CH- 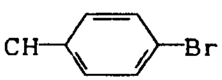 | |
| 111 | 2 | CH | $C_3H_7$ | $OCH_3$ | $OCH_3$ | -N=CH- 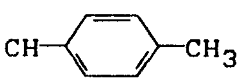 | |

**Tabelle 1** - Fortsetzung

| Bsp.-Nr. | n | A | $R^1$ | $R^2$ | $R^3$ | $R^4$ | Schmelzpunkt (°C) |
|---|---|---|---|---|---|---|---|
| 112 | 2 | N | $C_3H_7$ | $OCH_3$ | $CH_3$ | -N=CH- 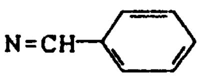 | |
| 113 | 2 | N | $C_3H_7$ | $CH_3$ | $OCH_3$ | -N=CH- 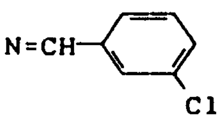 | |
| 114 | 2 | N | $C_3H_7$ | $OCH_3$ | $OCH_3$ | -N=CH- 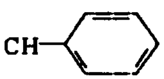 | |
| 115 | 2 | N | $C_3H_7$ | $OCH_3$ | $OCH_3$ | -N=CH- 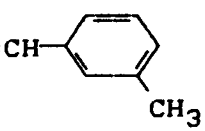 | |
| 116 | 2 | N | $C_3H_7$ | $OCH_3$ | $OCH_3$ | -N=CH- 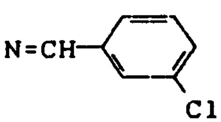 | |
| 117 | 2 | CH | $C_3H_7$ | $CH_3$ | $CH_3$ | -N=CH- 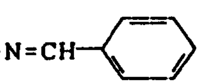 | |
| 118 | 2 | CH | $C_3H_7$ | $CH_3$ | $OCH_3$ | -N=CH- 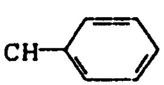 | |

<u>Tabelle 1</u> - Fortsetzung

| Bsp.-Nr. | n | A | $R^1$ | $R^2$ | $R^3$ | $R^4$ | Schmelzpunkt (°C) |
|---|---|---|---|---|---|---|---|
| 119 | 2 | CH | $C_3H_7$ | H | $CH_3$ | -N=CH- 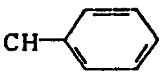 | |
| 120 | 1 | CH | $CH(CH_3)_2$ | $OCH_3$ | $OCH_3$ | -N=CH- 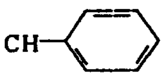 | |
| 121 | 1 | CH | $CH(CH_3)_2$ | $CH_3$ | $OCH_3$ | -N=CH- 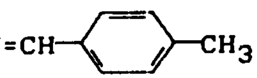 | |
| 122 | 1 | CH | $CH(CH_3)_2$ | H | $CH_3$ | -N=CH- 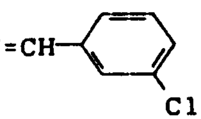 | |
| 123 | 1 | N | $CH(CH_3)_2$ | $CH_3$ | $OCH_3$ | -N=CH- 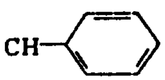 | |
| 124 | 1 | N | $CH(CH_3)_2$ | $OCH_3$ | $OCH_3$ | -N=CH- 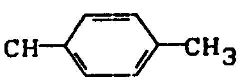 | |
| 125 | 1 | CH | $CH(CH_3)_2$ | $CH_3$ | $CH_3$ | -N=CH- 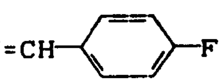 | |

**Tabelle 1** - Fortsetzung

| Bsp.-Nr. | n | A | $R^1$ | $R^2$ | $R^3$ | $R^4$ | Schmelzpunkt (°C) |
|---|---|---|---|---|---|---|---|
| 126 | 2 | CH | $CH(CH_3)_2$ | $CH_3$ | $OCH_3$ | -N=CH- 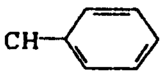 | |
| 127 | 2 | CH | $CH(CH_3)_2$ | $OCH_3$ | $OCH_3$ | -N=CH- 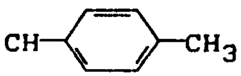 | |
| 128 | 2 | CH | $CH(CH_3)_2$ | H | $CH_3$ | -N=CH- 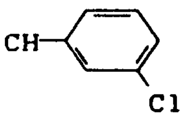 | |
| 129 | 2 | CH | $CH(CH_3)_2$ | $CH_3$ | $CH_3$ | -N=CH- 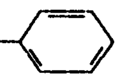 | |
| 130 | 2 | N | $CH(CH_3)_2$ | $CH_3$ | $OCH_3$ | -N=CH- 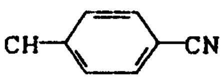 | |
| 131 | 2 | N | $CH(CH_3)_2$ | $OCH_3$ | $OCH_3$ | -N=CH- 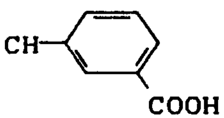 | |
| 132 | 1 | CH | $C_2H_5$ | $CH_3$ | $OCH_3$ | -N=CH- 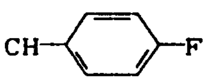 | |

<u>Tabelle 1</u> - Fortsetzung

| Bsp.-Nr. | n | A | $R^1$ | $R^2$ | $R^3$ | $R^4$ | Schmelzpunkt (°C) |
|---|---|---|---|---|---|---|---|
| 133 | 1 | CH | $C_2H_5$ | H | $CH_3$ | -N=CH- 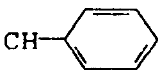 | 203 |
| 134 | 2 | CH | $C_2H_5$ | $CH_3$ | $OCH_3$ | -N=CH- 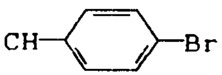 | |
| 135 | 2 | CH | $C_2H_5$ | H | $CH_3$ | -N=CH- 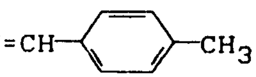 | |
| 136 | 1 | CH | $C_2H_5$ | $CH_3$ | $CH_3$ | -N=CH- 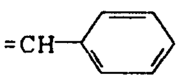 | 210 |
| 137 | 1 | N | $C_3H_7$ | $CH_3$ | $OCH_3$ | 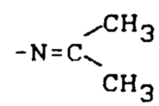 | 162 |
| 138 | 1 | CH | $C_2H_5$ | H | $CH_3$ | -N=CH- 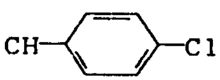 | 207 |
| 139 | 1 | CH | $C_2H_5$ | H | $CH_3$ | -N=CH- 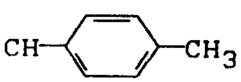 | 185 |

<u>Tabelle 1</u> - Fortsetzung

| Bsp.-Nr. | n | A | $R^1$ | $R^2$ | $R^3$ | $R^4$ | Schmelzpunkt (°C) |
|---|---|---|---|---|---|---|---|
| 140 | 1 | CH | $C_2H_5$ | H | $CH_3$ | 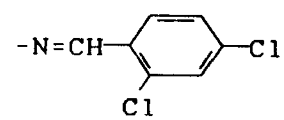 | 205 |
| 141 | 1 | CH | $C_3H_7$ | H | $CH_3$ | 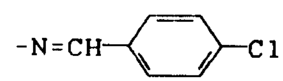 | 215 |
| 142 | 1 | CH | $C_3H_7$ | H | $CH_3$ | 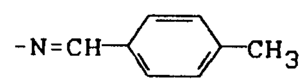 | 203 |
| 143 | 1 | CH | $C_3H_7$ | H | $CH_3$ | 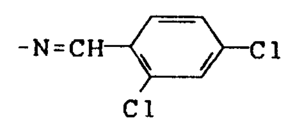 | 180 |
| 144 | 1 | N | $C_3H_7$ | $OCH_3$ | $OCH_3$ | 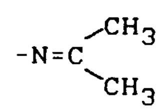 | 153 |

<u>Ausgangsstoffe der Formel (II)</u>

<u>Beispiel (II-1)</u>

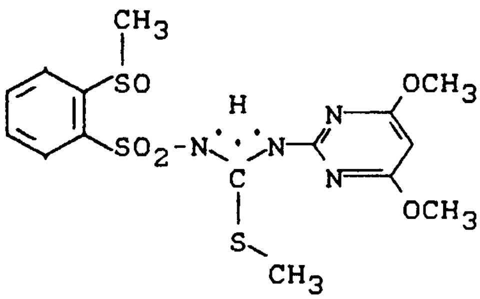

20,7 g (0,05 Mol) N'-(4,6-Dimethoxy-pyrimidin-2-yl)-N''-(2-methylthio-phenylsulfonyl)-S-methyl-isothioharnstoff werden mit 100 ml Essigsäure verrührt und zu dieser Mischung werden bei 20 °C bis 30 °C 6,8 g einer 30%igen wäßrigen Lösung von Hydrogenperoxid (0,06 Mol $H_2O_2$) tropfenweise gegeben. Das Reaktionsgemisch wird dann 5 Stunden bei 60 °C bis 70 °C gerührt; das Rühren wird noch 15 Stunden bei 20 °C fortgesetzt und anschließend wird das kristalline Produkt durch Absaugen isoliert.

Man erhält 19,35 g (90% der Theorie) N'-(4,6-Dimethoxy-pyrimidin-2-yl)-N''-(2-methylsulfinyl-phenylsulfonyl)-S-methyl-isothioharnstoff vom Schmelzpunkt 117 °C.

Anwendungsbeispiele:

In den folgenden Anwendungsbeispielen wird die nachstehend aufgeführte Verbindung als Vergleichssubstanz herangezogen:

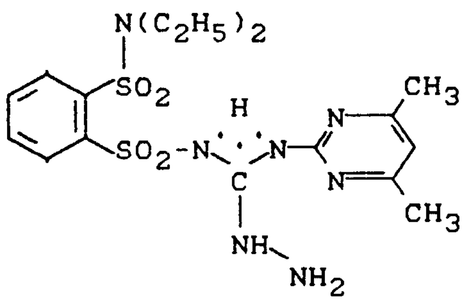

(A)

N'-(4,6-Dimethyl-pyrimidin-2-yl)-N''-amino-N'''-(2-diethylaminosulfonyl-phenylsulfonyl)-guanidin (bekannt aus EP-A 173319, Bsp. 40).

Die Formeln der für die Anwendungsbeispiele herangezogenen erfindungsgemäßen Verbindungen sind - mit der Numerierung der Herstellungsbeispiele (Bsp.-Nr. aus Tabelle 1) nachstehend einzeln aufgeführt.

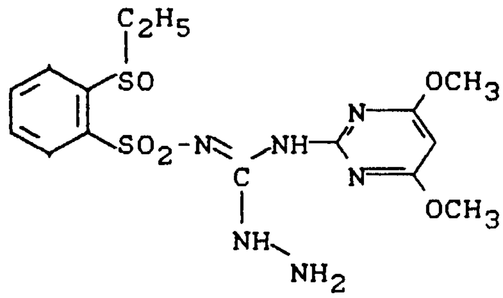

(1)

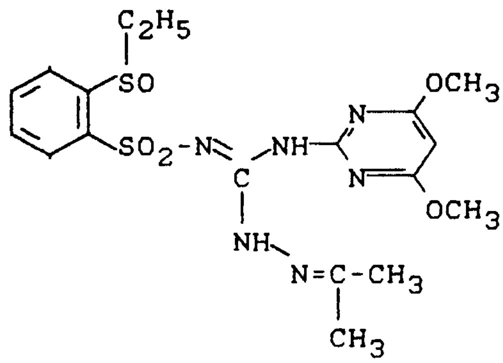

(3)

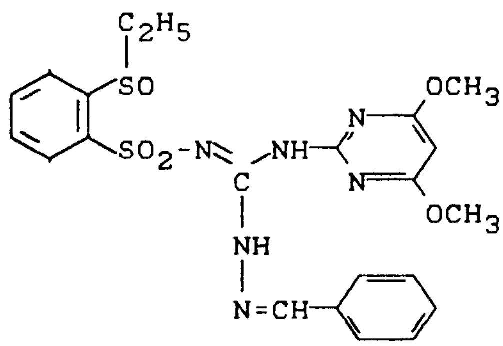

(4)

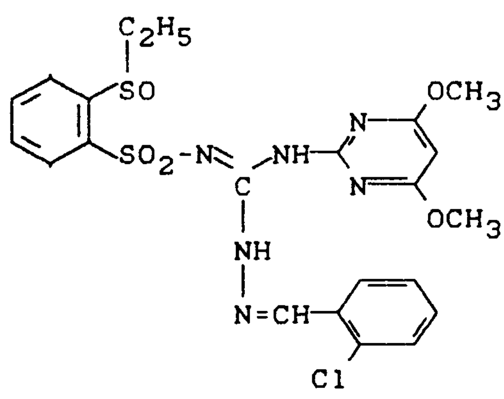

(5)

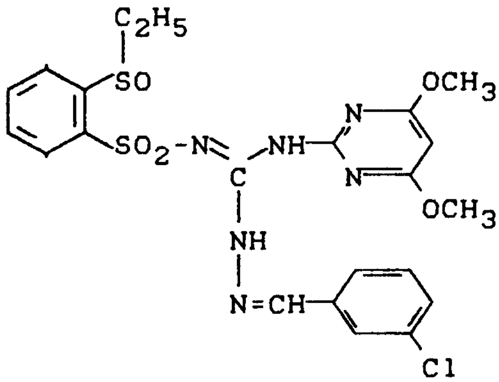

(2)

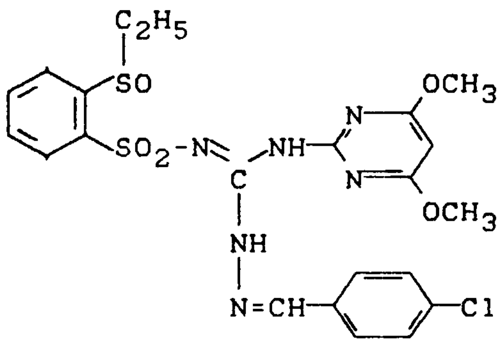

(6)

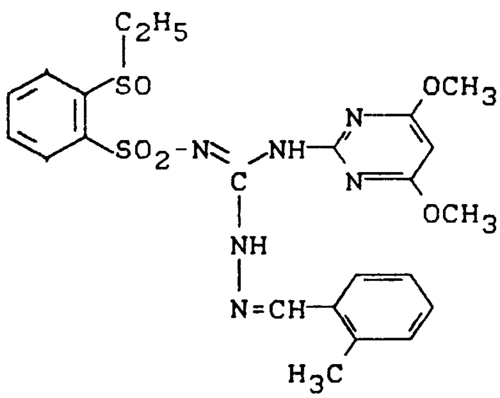

(7)

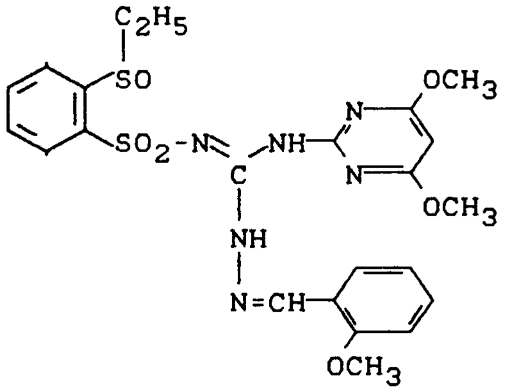

(8)

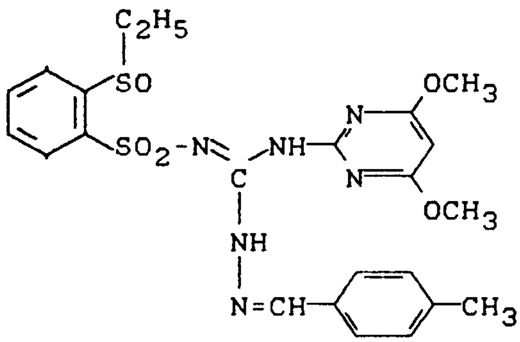

(9)

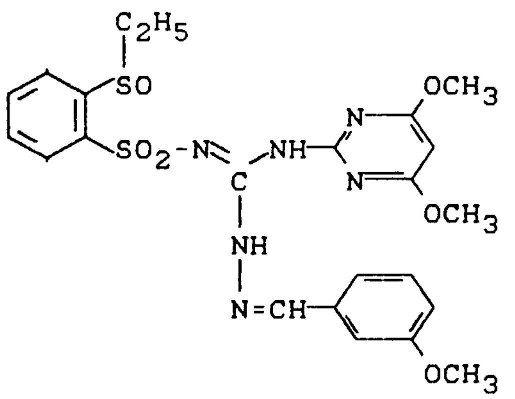

(10)

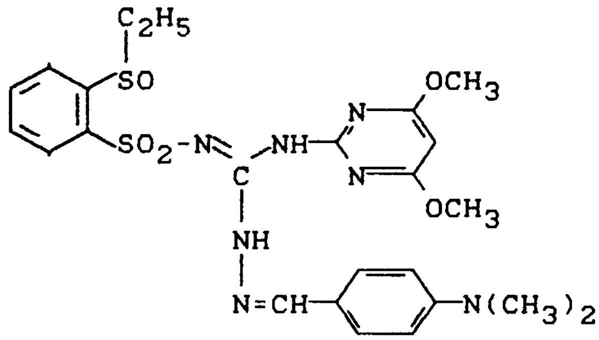

(12)

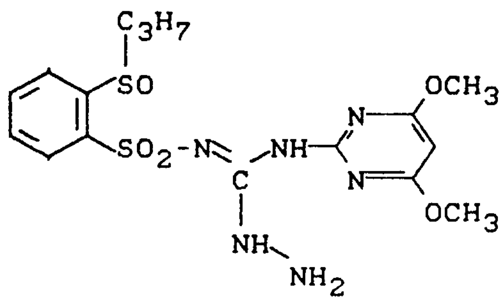

(13)

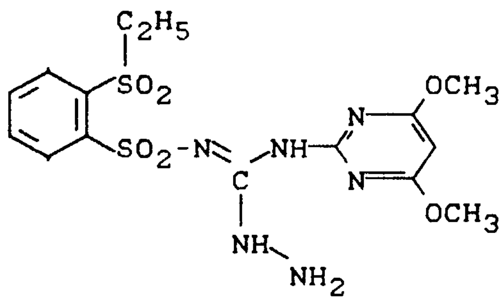

(22)

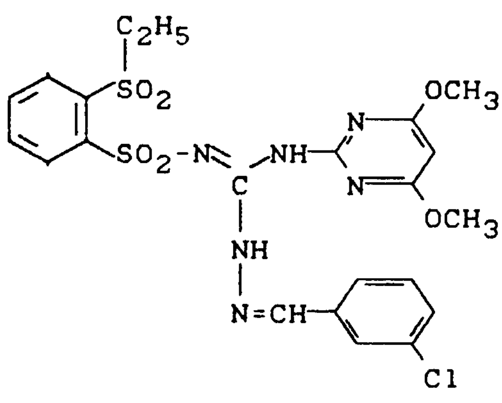

(26)

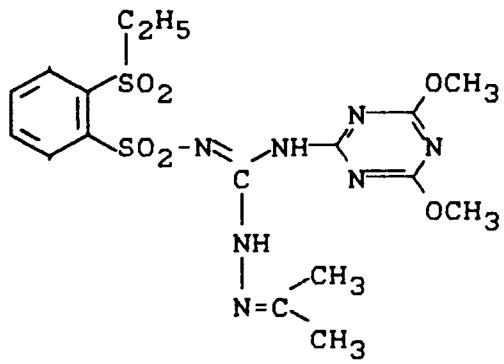

(35)

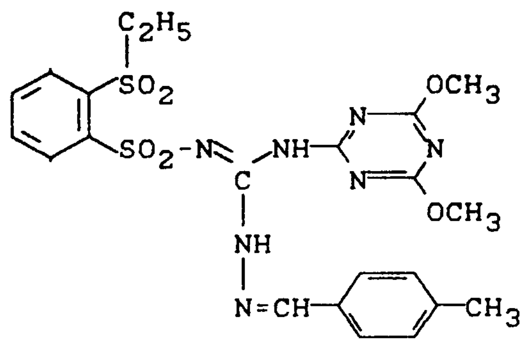

(39)

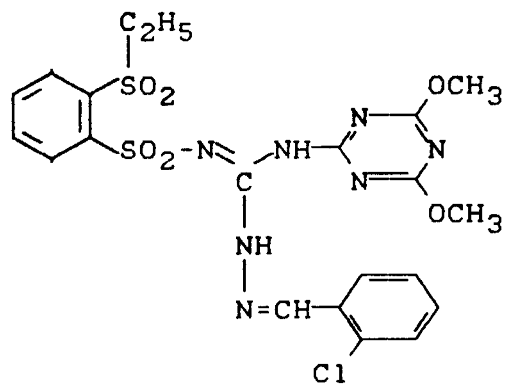

(42)

Beispiel A

Post-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

Eine deutliche Überlegenheit in der Wirksamkeit gegenüber dem Stand der Technik bei guter Verträglichkeit gegenüber Nutzpflanzen, wie z.B. Mais und Weizen, zeigen in diesem Test z.B. die Verbindungen gemäß folgender Herstellungsbeispiele: 1, 2, 3, 4, 5, 6, 7, 8, 9, 10, 12, 13, 22, 26, 35, 39 und 42.

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten;

0 % = keine Wirkung (wie unbehandelte Kontrolle)
100 % = totale Vernichtung

Eine deutliche Überlegenheit in der Wirksamkeit gegenüber dem Stand der Technik bei guter Verträglichkeit gegenüber Nutzpflanzen, wie z.B. Weizen, Soja und Baumwolle, zeigen in diesem Test z.B. die Verbindungen gemäß folgender Herstellungsbeispiele: 1 und 22.

**Patentansprüche**

**1.** Sulfonylguanidinoazine der allgemeinen Formel (I)

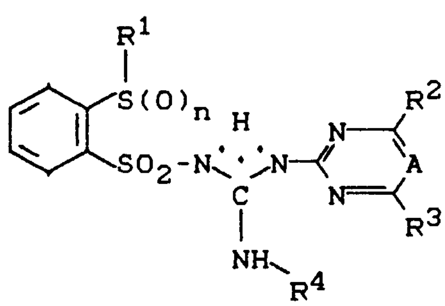

(I)

in welcher

n für die Zahlen 1 oder 2 steht,

A für Stickstoff oder eine C-X-Gruppierung steht, wobei X für Wasserstoff oder Halogen steht,

$R^1$ für Alkyl mit mindestens zwei Kohlenstoffatomen steht,

$R^2$ und $R^3$ gleich oder verschieden sind und für Wasserstoff, Halogen, Alkyl, Halogenalkyl, Alkoxyalkyl, Alkoxy, Halogenalkoxy, Alkylthio, Alkylamino oder Dialkylamino stehen,

und

$R^4$ für Amino oder die Gruppierung

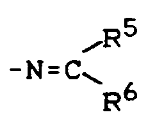

steht,

worin

$R^5$ für Wasserstoff oder für jeweils gegebenenfalls substituiertes Alkyl, Aryl oder Aralkyl steht,

$R^6$ für Wasserstoff oder für jeweils gegebenenfalls substituiertes Alkyl, Alkenyl, Alkadienyl, Alkinyl, (Hetero)Aryl, Aralkyl, Aralkenyl, Alkoxy, Alkoxycarbonyl oder Dialkylamino steht, oder zusammen mit $R^5$ für gegebenenfalls substituiertes Alkandiyl steht.

**2.** Verfahren zur Herstellung von Sulfonylguanidinoazinen der allgemeinen Formel (I) gemäß Anspruch 1,

dadurch gekennzeichnet, daß man

(a) für den Fall, daß $R^4$ für Amino steht und n, A, $R^1$, $R^2$ und $R^3$ die oben angegebenen Bedeutungen haben,

Sulfonyliso(thio)harnstoffe der allgemeinen Formel (II)

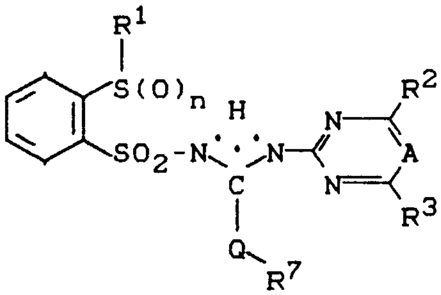

(II)

in welcher

n, A, $R^1$, $R^2$ und $R^3$ die oben angegebenen Bedeutungen haben,

Q für Sauerstoff oder Schwefel steht und

$R^7$ für jeweils gegebenenfalls substituiertes Alkyl, Aralkyl oder Aryl steht,

mit Hydrazin oder einem Hydrazin-Wasser- oder Hydrazin-Säure-Addukt,

gegebenenfalls in Gegenwart eines Säureakzeptors und gegebenenfalls in Gegenwart eines Verdünnungsmittels umsetzt, oder daß man

(b) für den Fall, daß $R^4$ für die Gruppierung

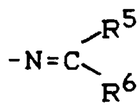

steht und n, A, $R^1$, $R^2$, $R^3$, $R^5$ und $R^6$ die oben angegebenen Bedeutungen haben,

Sulfonylguanidinoazine der allgemeinen Formel (I), in welcher $R^4$ für Amino steht und n, A, $R^1$, $R^2$ und $R^3$ die oben angegebenen Bedeutungen haben,

mit Carbonylverbindungen der allgemeinen Formel (III)

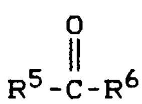

(III)

in welcher

$R^5$ und $R^6$ die oben angegebenen Bedeutungen haben,

oder mit N,N-Dialkyl-carbonsäureamid-acetalen bzw. -ketalen

gegebenenfalls in Gegenwart eines Kondensationshilfsmittels und gegebenenfalls in Gegenwart eines Verdünnungsmittels umsetzt.

**3.** Herbizide Mittel, gekennzeichnet durch einen Gehalt an mindestens einem Sulfonylguanidinoazin der Formel (I) gemäß Anspruch 1.

**4.** Verfahren zur Bekämpfung von Unkräutern, dadurch gekennzeichnet, daß man Sulfonylguanidinoazine der Formel (I) gemäß Anspruch 1 auf Unkräuter und/oder ihren Lebensraum einwirken läßt.

**5.** Verwendung von Sulfonylguanidinoazinen der Formel (I) gemäß Anspruch 1 zur Bekämpfung von Unkräutern.

**6.** Verfahren zur Herstellung von herbiziden Mitteln, dadurch gekennzeichnet, daß man Sulfonylguanidinoazine der Formel (I) gemäß Anspruch 1 mit Streckmitteln und/oder oberflächenaktiven Mitteln vermischt.

Europäisches Patentamt

# EUROPÄISCHER RECHERCHENBERICHT

Nummer der Anmeldung

EP 92 10 4972

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | EP-A-0 343 462 (BAYER)<br>* Ansprüche * | 1-6 | C07D239/52<br>C07D239/47<br>C07D239/42<br>C07D251/46<br>C07D251/42<br>A01N47/44 |
| A,D | EP-A-0 173 319 (BAYER AG)<br>* Ansprüche 1,3-5 * | 1,3-6 | |
| A | EP-A-0 414 067 (BAYER AG)<br>* Ansprüche 1,3-6 * | 1,3-6 | |
| P,A | EP-A-0 431 270 (BAYER AG)<br>* Tabelle 3, Beispiele 312, 313, 317, 1327-1337, 1340, 1341, 1376, 1405, 1408, 1527-1531; Ansprüche * | 1-6 | |
| D | & DE-A-3 933 792 | | |
| D | & DE-A-4 017 460 | | |

RECHERCHIERTE SACHGEBIETE (Int. Cl.5)

C07D
A01N

Der vorliegende Recherchenbericht wurde für alle Patentansprüche erstellt

| Recherchenort | Abschlußdatum der Recherche | Prüfer |
|---|---|---|
| BERLIN | 11 JUNI 1992 | VAN AMSTERDAM L. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)